# EUROPEAN PATENT APPLICATION

(11) **EP 2 851 101 A1**
(43) Date of publication of application: **25.03.2015**
(21) Application number: 12875147.6
(22) Date of filing: 28.05.2012
(51) Int. Cl.: A61M 5/32, A61M 5/50

(54) **NEEDLE BASE RETRACTABLE-TYPE SAFE SELF-LOCKING-TYPE SELF-DESTRUCTING SYRINGE**

(30) Priority: 27.04.2012 CN 201210127996
(71) Applicant: Zhang, Linfeng, Wenzhou, Zhejiang 325024 (CN)
(72) Inventor: Zhang, Linfeng, Wenzhou, Zhejiang 325024 (CN)
(74) Representative: Gualeni, Nadia
(86) International application number: PCT/CN2012/000739
(87) International publication number: WO 2013/159254

(57) **Abstract**

A retractable-type safe self-locking-type self-destructing syringe, which comprises a syringe barrel body (6). A plunger (7) is arranged in the syringe barrel body (6). A needle base is arranged in a barrel neck at an end part of the syringe barrel body (6). A sealing ring (3) is sleeved on the outer wall of the needle base. A head part for needle replacement is formed by the upper part of the needle base. A flexible support engaging piece (4) is arranged within a cavity at the lower part of the needle base. A locking-clutching mechanism matching the end part of the plunger (7) is arranged within the flexible support engaging piece (4). The locking-clutching mechanism is provided with a flexible characteristic. Also, a flexible support limiting mechanism matching the inner wall of the cavity of the syringe barrel body (6) is formed by the exterior of the flexible support engaging piece (4). A clutching part matching the locking-clutching mechanism is formed by the end part of the plunger (7).

## Description

### Technical field

The present invention relates to a medical instrument technical field, and in particular, to a needle base retractable-type safe self-locking-type self-destructing syringe, especially to a mechanical connection structure thereof.

### Background art

A document 1 application number of which is 200910029167.X and the applicant of which is Ligang, ZHOU, the application date of which is January 7, 2009, the publication number of which is CN101474443A, discloses a safe self-destruction medical syringe in which a movable needle base and a outer jacket are respectively provided thereon with a protrusion portion and a clamping boss cooperating with the protrusion portion lapping over each other. The movable needle base is provided with a hanging hook to be penetrated into a chamber containing injection fluid. A sealing plug is provided with a restriction inclination surface for the hanging hook and with a fastening cavity for connecting the sealing plug to the hanging hook as an integral part. The diameter of the outer jacket on one side of the hanging hook is larger than the outer diameter of the movable needle base and on the other side of the hanging hook the movable needle base is restricted therein.

A document 2 the application number of which is 200610049044.9 and the applicant of which is Zuoqian LIN and the application date of which is January 12, 2006 and the publication number is CN1803212A discloses a safe self-destruction medical syringe which is provided under the cone seat with two through grooves which are symmetrical about a central axis and in the middle of which is provided with two bosses which are symmetrical about the central axis and clamp corresponding recesses. The cone seat is provided on the bottom of the inner hole with a recess; the support seat is provided in the front end with a plurality of elastic support claws able to clamp the boss on the head of the core bar and on the middle part of the outer side with a protruded lip.

There are various self-destruction syringe so far and many safety syringe but the parts thereof are designed complicatedly and processed hard as well as assembled inconveniently so as to result in a high cost; the tip of the needle has an insufficient needle withdrawal resistance and hence easily is withdrawal into the outer jacket to cause medicine fluid to be leaked. On the other hand, the needle withdrawal resistance is increased intentionally so that the pullback resistance is also increased negatively so as to cause the user inconvenient; when using it, the needle base is susceptible to rotate and it requires much force to activate the self-destruction device so as to result in trembling needle tube so that the patient suffers; the pushing prevention part avoiding wrong operation requires two hands' work and collection of pushing prevention parts picked by hands. Therefore, it is necessary to improve current safety self-locking syringe so that the cost of manufacturing and assembling the relevant parts is reduced and the pulling resistance is reduced while the needle withdrawal resistance is increased and meanwhile the needle base is ensured not to rotate and the needle tube cannot tremble when activation of the self-destruction means. In addition, the needle tip is self-locked within the inner cavity of the outer jacket and a protection plate is provided for single hand operation to prevent wrong operation during transporting and holding it in order to realize a mass production and a clinical safety utilization.

### Summary of the invention

The purpose of the invention is to propose a needle base retractable safe self-locking type self-destruction syringe in which the needle base can be assembled with the outer jacket in any angle and the needle base is shaped in a simple and efficient way with a high needle withdrawal resistance and low cost of self-locking means. In addition, the needle of such syringe is secured stably by means of tooth hub and tooth sheath to prevent rotation and hence the force for activation of the self-destruction means is low and the needle tube cannot tremble so as to cause the syringe to be more safe and reliable. Meanwhile the self-destruction structure between the core bar and syringe tube body positively avoids the needle to be pushed out second time and thereby the safe operation of the syringe is really realized to overcome the disadvantages and deficiency of the prior art.

In order to realize the above purposes, the technical solutions of the present invention defines as follows: a needle base retractable type safe self-locking type self-destruction syringe, comprising a syringe tube body which is provided therein with a core bar over which a piston fits adjacent to the end of the core bar , a needle base provided in the tube journal at the end of the syringe tube body, a sealing ring fitting over the outer wall of the needle base, characterized in that an interchangeable needle head being formed in the upper portion of the needle base and an elastic supporting clamping part being provided in the lower cavity of the needle base and being provided with a locking engagement structure to cooperate with the end of the core bar, the locking engagement structure having a nature of elasticity, the elastic supporting clamping part is provided on the outer portion with an elastic supporting position-limiting structure to cooperate with the inner wall of the inner cavity of the syringe tube body, the end of the core bar forming an engagement part to engage with the locking engagement structure.

Another technical solution of the invention lies in that a needle base retractable type safe self-locking type self-destruction syringe, comprising a syringe tube body which is provided therein with a core bar over which a piston fits adjacent to the end of the core bar, a needle base provided in the tube journal at the end of the syringe tube body, a sealing ring fitting over the outer wall of the needle base, characterized in that an elastic supporting clamping part being provided in the lower cavity of the needle base and being provided with a locking engagement structure to cooperate with the end of the core bar, the locking engagement structure having a nature of elasticity, the elastic supporting clamping part is provided on the outer portion with an elastic supporting position-limiting structure to cooperate with the inner wall of the inner cavity of the syringe tube body, the end of the core bar forming an engagement part to engage with the locking engagement structure.

The present invention discloses a needle base retractable safe self-locking self-destruction syringe whose positive effects are that the needle base and the outer jacket can be directly embedded one in another at any direction along the whole circumference; the needle withdrawal resistance is applied to the tooth hub conical seat and the position limitation of the clamping claw can supply sufficient needle withdrawal resistance; during being pulled back, the clamping claw can reduce pulling back resistance in the opposite direction by deformation of the clamping claw; the locking engagement structure for cooperating the elastic supporting clamping part with the end of the core bar is of elastic nature and thereby during clinical injection, the process of penetration of the end of the core bar into the locking engagement structure is not apparent; after penetration of the end of the core bar the locking engagement structure loses a nature of inward elasticity so that the pulling back engagement can be realized. The self locking structure between the core bar and the outer jacket is produced in a simple way but reaches the self-locking effect and thereby the safe operation of the product is improved efficiently. The whole design of the syringe is reasonable and it works stably. Compared to the prior art, it posses prominent substantial features and notable developments.

### Brief descriptions of the drawings

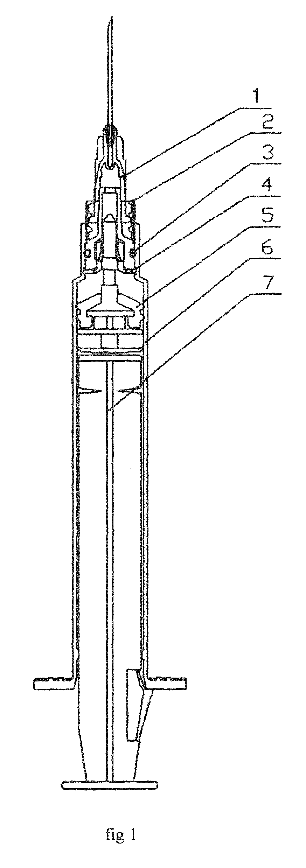

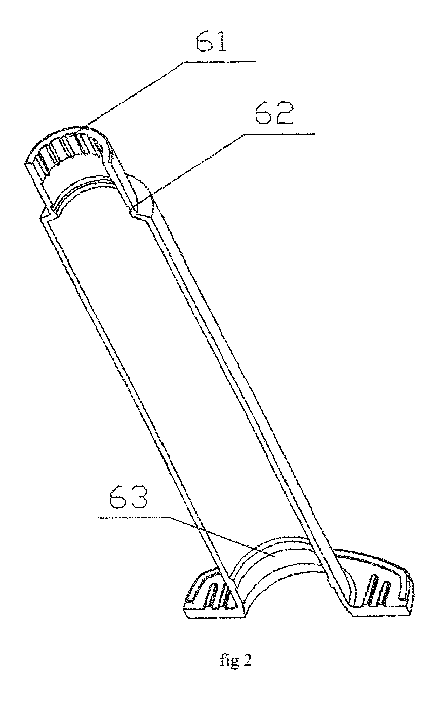

Fig.1 is a cut view of a planar structure of a first embodiment of the invention;
Fib. 2 is a cut view of a perspective structure of the tube body of the syringe;

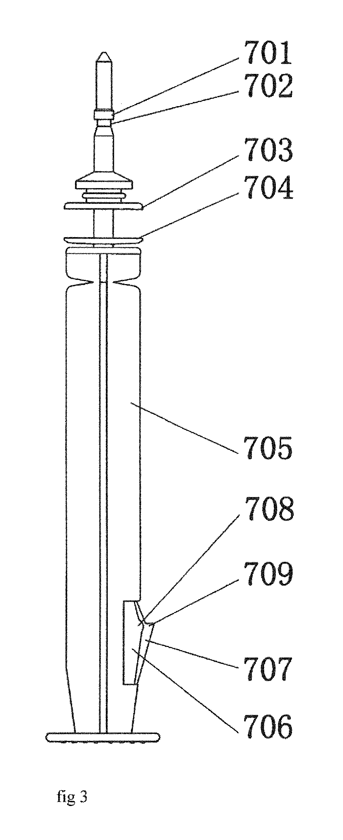

Fig3. is a cut view of the structure of the core bar;

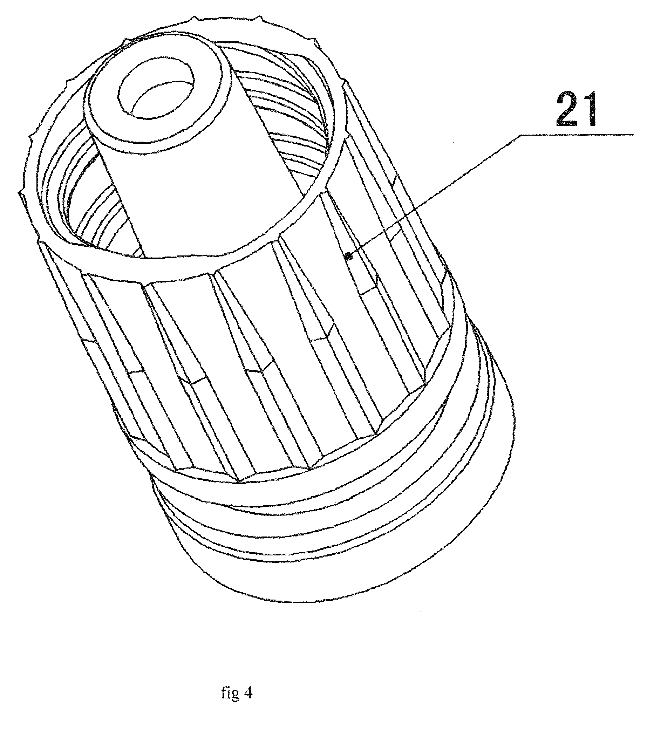

Fig4. is a schematic view of the perspective structure of the tooth hub conical seat;

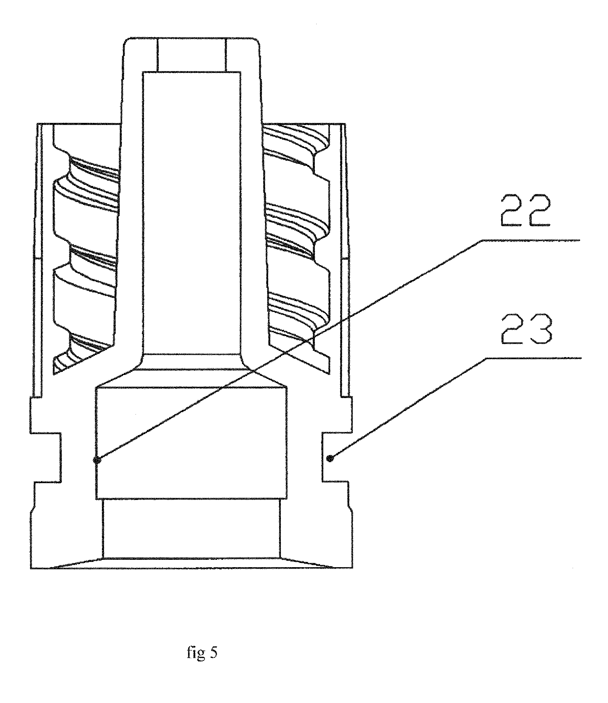

Fig.5 is a cut view of the planar structure of the tooth hub conical seat;

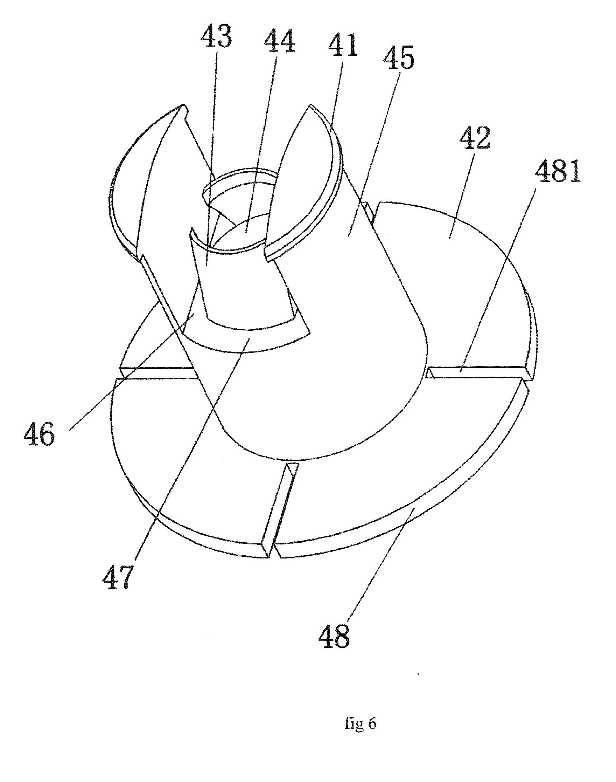

Fig. 6 is a schematic view of the perspective structure of the elastic supporting clamping part;
Fig. 7 is a schematic view of a primitive state of the first embodiment of the syringe;
Fig. 8 is a cut view of the planar structure of the first embodiment of the syringe under suction of fluid;
Fig.9 is a cut view of the planar structure of the first embodiment of the syringe under pushing of fluid;
Fig.10 is a cut view of the planar structure of the first embodiment of the syringe under completion of injection of fluid;
Fig.11 is a cut view of the planar structure of the first embodiment of the syringe under separation of the clamping claw from the withdrawal prevention groove;
Fig.12 is a cut view of the first embodiment of the syringe under self-locking of the core bar;
Fig.13 is a cut view of the first embodiment of the syringe under rupture of the core bar;

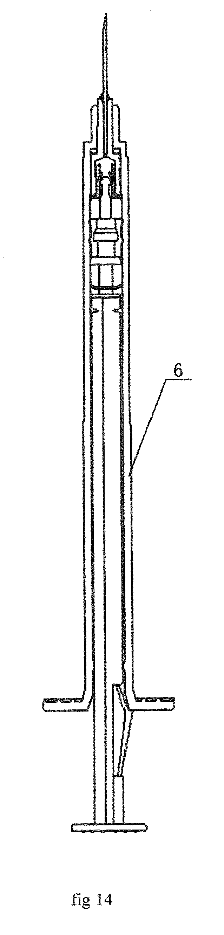

Fig.14 is a cut view of the planar structure of the second embodiment of the present invention;

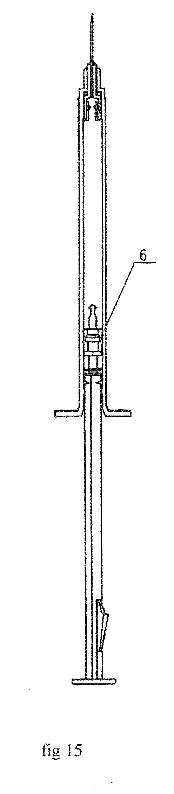

Fig.15 is a cut view of the planar structure of the second embodiment of the syringe under suction of the fluid;

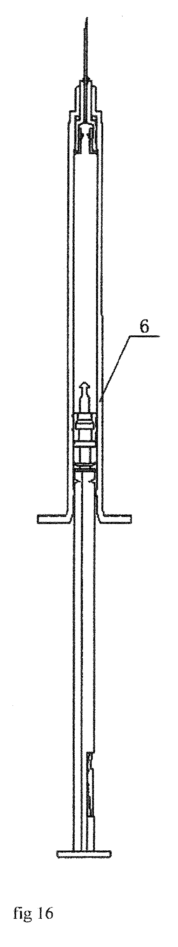

Fig.16 is a cut view of the planar structure of the second embodiment of the syringe under pushing of the fluid;

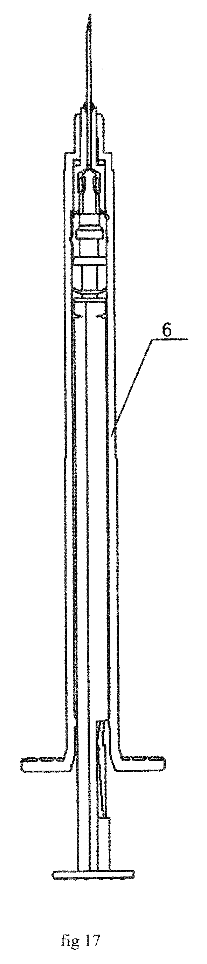

Fig.17 is a cut view of the planar structure of the second embodiment of the syringe under completion of injection of the fluid;

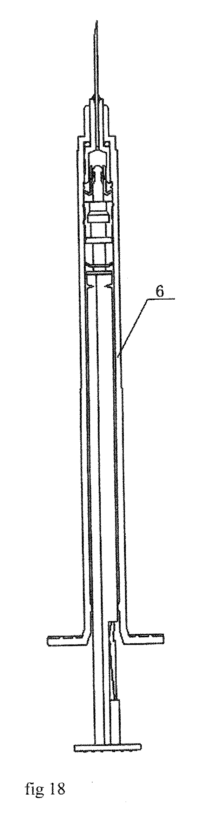

Fig.18 is a cut view of the planar structure of the second embodiment of the syringe under separation of the clamping claw from the withdrawal prevention groove;

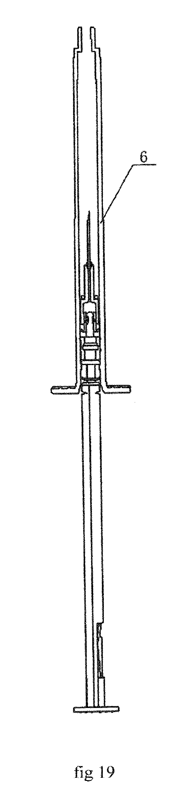

Fig.19 is a cut view of the second embodiment of the syringe under self-locking of the core bar;

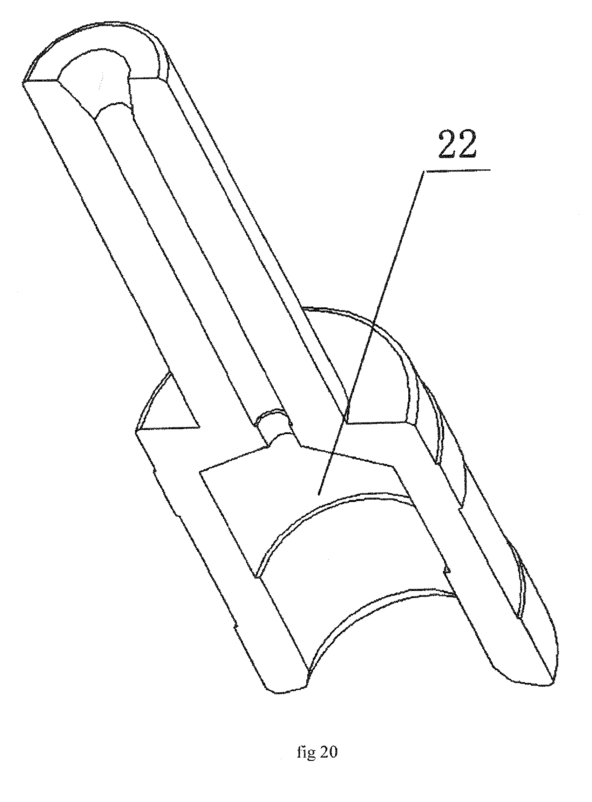

Fig.20 is a cut view of the needle base of the second embodiment of the syringe under suction of the fluid; and

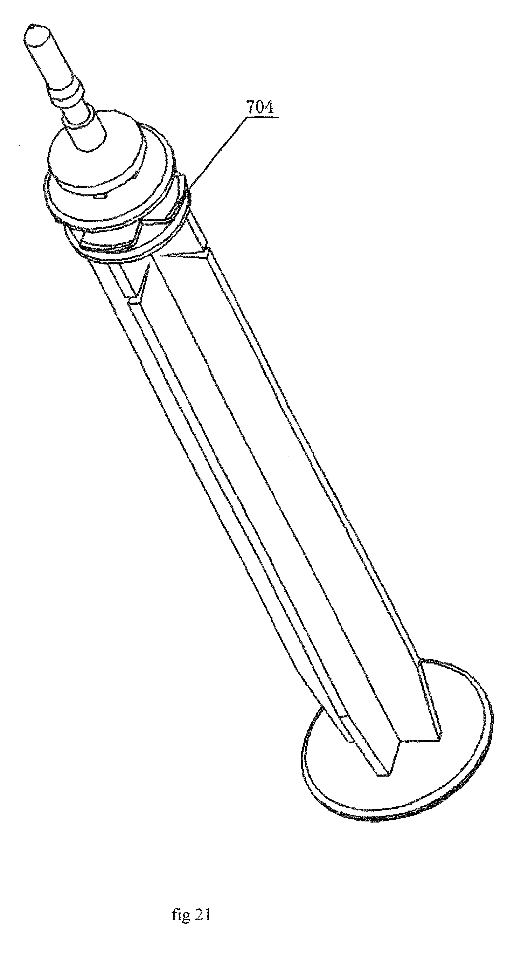

Fig.21 is a perspective of the core bar of the syringe.

### Best carried-out examples

Referring to the drawings, the present invention is more detailed described.

The invention relates to a needle base retractable safety self-destruction syringe, it comprises a tube body 6 of the syringe. The syringe tube body 6 is provided therein with a core bar 7 over which a piston fits adjacent to its end. The syringe tube body 6 is provided in the tube journal on the end with a needle base which has an outer wall over which a sealing ring 3 fits. It distinguishes from the prior art in that an interchangeable needle head is formed in the upper portion of the needle base, and the needle base is provided on the lower cavity with an elastic supporting clamping part 4 which is provided therein with a blocking engagement structure cooperating with the end of the core bar and which has a nature of elasticity. The elastic supporting clamping part 4 is provided on the outer portion with an elastic supporting position limiting structure to cooperate with the inner wall of the inner cavity of the syringe tube 6 and the core bar is provided on the end with an engagement part to cooperate with the locking engagement structure.

Another embodiment of the invention defines in that a needle base retractable safety self-blocking and self-destruction syringe comprising a syringe tube body 6 in which is provided a core bar 7 over which a piston 5 fits at the position adjacent to the end. The syringe tube 6 is provided within the tube journal of the end with a needle base which has an outer wall over which a sealing ring fits. It distinguishes from the prior art in that the needle base is provided on the lower cavity with an elastic supporting clamping part 4 which is provided therein with a blocking engagement structure to cooperate with the end of the core bar and having a nature of elasticity. The elastic supporting part 4 is provided on the outer portion with an elastic supporting position-limiting structure to cooperate with the outer wall of the inner cavity of the syringe tube 6 and the core bare is provided on the end with an engagement part to cooperate with the locking engagement structure.

In the particular realization of the present invention, the exchangeable needle has a head which is a conical seat with a guidance tooth hub 2, which has a screwed opening able to engage with the injection needle 1 of international standard of 6:100 so as to interchange the needle.

In the particular realization of the invention, the needle base is provided on the inner wall of the lower cavity with an annular internal recess 22 and on the outer wall with an annular external recess 23. The sealing ring 3 is provided in the annular external recess 23 so as to permit a sealing effect between the needle base and the inner wall of the tube body.

In the particular realization of the present invention, the conical seat 2 of the guidance tooth hub is provided on the outer wall with tooth hubs 21 distributed annually evenly and with guidance function. The guidance structure can ensure the tooth hub conical seat to accurately assemble into the syringe tube body in any direction so as to realize a completely automatic production with a low cost during assembling.

In the particular realization of the invention, the elastic supporting clamping part 4 is in form of an approximate hollow cylinder and is provided on the upper portion with two symmetrical elastic deformation wings 45 on whose upper external edge an annular protrusion lip 41 to cooperate with the annular internal recess 22 is formed, between which a cutout 44 is formed. The cutout 44 is provided on the lower portion with an elastic supporting clamping part 4 which is provided on the upper surface with two symmetrical curved bosses 47 each of which is provided with a ratchet plate 43. The two ratchet plates 43 are symmetrical to each other and the extending lines of the upper ends of the two ratchet plates 43 form an angle. A gap is formed between two sides of the ratchet plates 43 and the inner side of the elastic deformation wings 45. The two opposite ratchet plates 43 constitute the locking engagement structure and the inner side of the ratchet plate 43 can expand towards outer side when being forced. The ratchet plate 43 is of elastic nature. The elastic supporting clamping part 4 is provided on the lower portion with a circular clamping plate 48 where are provided a plurality of broken gaps 481. In a particular realization, there can be four broken gaps 481 in which the adjacent ones form an angle of 90 degree and which are distributed according to a cross form. The circular clamping plate 48 is divided into four separate clamping claw 42 by four broken gaps 481 distributed by the cross form.

In the particular realization of the invention, the syringe tube body 6 is provided on the inner wall of the tube journal at the end with a certain amount of tooth sleeves which corresponds to the amount of the tooth hubs. The mechanism of engagement between the tooth hub and tooth sleeve can prevent the injection needle from rotation when injection and hence avoid any pain the patient may suffer due to it. The tooth sleeve 61 is provided on the inner wall of the tube journal on the lower portion with an annular withdrawal prevention groove 62 to cooperate with the clamping claw 42. The syringe tube body 6 is provided on the inner wall on the lower portion with a self-locking ring 63 in form of protrusion.

In the particular realization of the invention, the core bar 7 has an engagement part at the end and which is an annular boss 701 whose outer diameter is larger than distance between the top ends of the two ratchet plates 43. An annular clamping groove 702 is formed at the core bar at the lower portion of the annular boss 701 and a circular position-limiting plate 703 is formed at the core bar on the lower portion of the annular clamping groove 702 and some wing blades 704 distributed divergently are provided at the core bare on the lower portion of the circular position-limiting plate 703. The cooperation between the wing blades 704 and self-locking ring in the syringe tube body 6 enables the core bar to be incapable of forward pushing and backward pulling. The piston 5 fits over the core bar 7 between the annular clamping groove 702 and the circular position-limiting plate 703 and the piston 5 is secured by the circular position-limiting plate 703.

In the particular realization of the invention, the annular boss 701 has a diameter larger than the distance between two ratchet plates 43 and thus the annular boss 701 on the end of the core bar is on the ends of the two ratchet plates 43 when the core bar is pulled back so that the clamping and locking effect of the core bar and the needle base is realized.

In the particular realization of the invention, the core bar 7 is provided with a reinforced flanges 705 distributed by a cross form and in which one is provided with a cutout 706 which also is provided with a protection plate 707 both ends of which form a junction with points of the two inner walls of the cutout. The protection plate 707 is provided on the part close to the middle with a bending portion 708 which is provided with a protrusion 709. When completion of the assembling, the protrusion of the protection plate just abuts against the lower outer edge of the syringe tube body 6 and when utilization of the syringe, the protection plate is pressed towards the inner side along the bending portion and is bent towards the inner side so that the protrusion is displaced towards the inner side till it leaves the lower outer edge of the syringe tube body 6 and therefore the syringe can be used normally. Such structure can prevent efficiently the syringe from wrong operation and can activate the safe structure inside so as to cause the syringe to be disposed of.

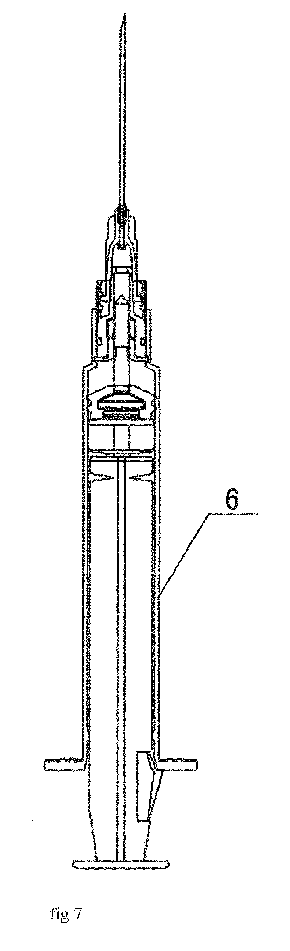

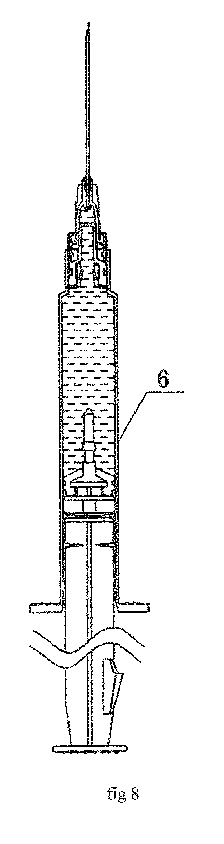

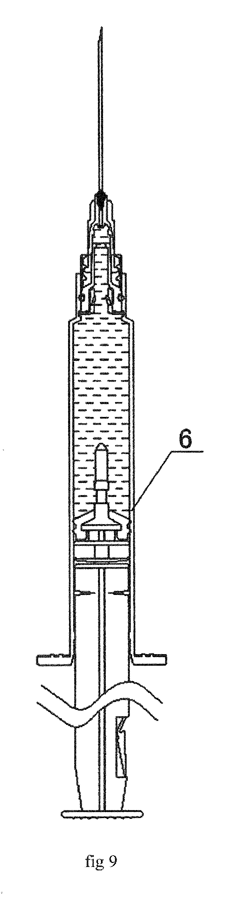

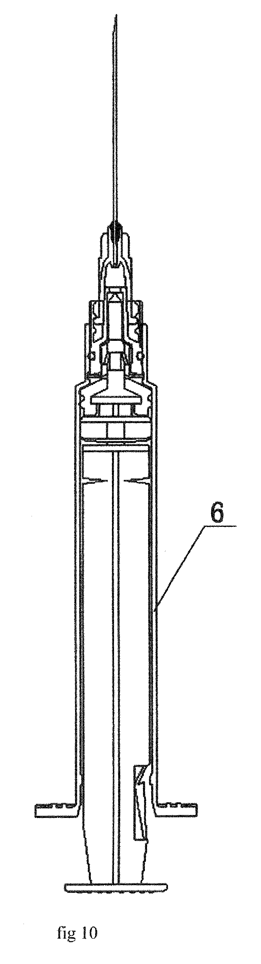

Figs 7-13 illustrate the whole utilization process of the invention. Fig 8 is a cut view of the planar structure of the completion of suction of the fluid. Before pushing the fluid the user presses down the protection plate first so as to alleviate the resistance to the forward pushing of the core bar. Fig 9 is a cut view of the planar structure of the pushing the fluid. During the pushing of the fluid, the needle withdrawal resistance is produced by the outer ring of the clamping claw of the elastic supporting clamping part and the withdrawal prevention groove of the syringe tube body, which brings about a proper needle withdrawal resistance and realizes a balancing compromise between the needle withdrawal resistance and the pull resistance. When the fluid is injected completely, the shrinkage inclination surface on the top end of the core bar will brace two ratchet claws of the elastic supporting clamping part towards two lateral sides smoothly. Once it goes over the upper end of the ratchet claw, the ratchet claw is clamped into the clamping groove of the core bar and hence the injection is completed as seen in fig 10.

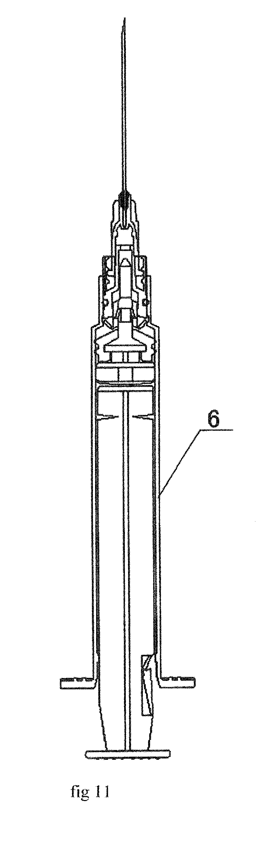

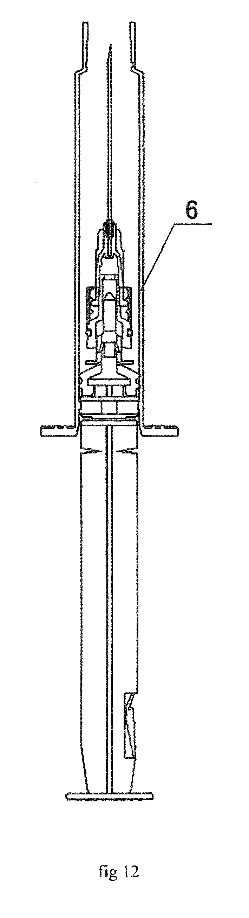

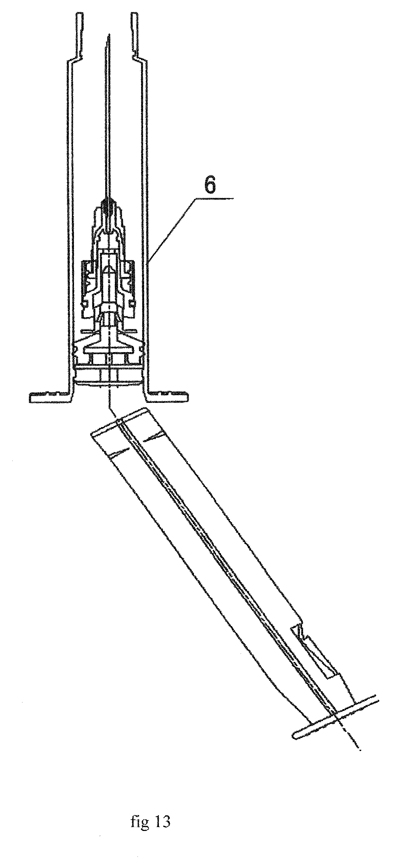

The core bar is pulled back after completion of the injection as seen in fig 11, the engagement between the clamping groove of the core bar and the ratchet claw will carry the elastic supporting part together to move downward and meanwhile the clamping claw of the elastic supporting clamping part is being bent so that the outer diameter of the clamping claw is becoming narrower and so is off the withdrawal prevention groove of the outer jacket. When the core bar is further pulled downward, and when the lower end face of the annular protrusion lip of the elastic supporting clamping part comes into contact with the lower end face of the inner recess of the tooth hub conical seat, the core bar carries the whole needle base comprising normal injection needle to move till the wing blades of the core bar go over self-locking ring of the syringe tube body and the self-locking ring of the syringe tube body is blocked between the circular position-limiting plate and the wing blades of the core bar stably and at the same time the core bar cannot backward pulling nor forward pushing. As seen in fig 12, the whole head of the needle is retraction inward of the inside of the needle tube and the safety self-locking is realized. As seen in fig 13, finally a small force is used for the self-destruction point so that the core bar is ruptured or broken and hence the head of the core bar is destructed inside of the jacket.

The above is a descriptions in detail of the present invention, in connection with the detailed preferably embodiments, which cannot be deemed to limit the scope of the invention by the whole embodiments of the present invention. With respect to the present invention, those skilled in the art, without being away from the principle of the present invention, can devise some solutions by means of deduction and/or replacement, which should also be regarded as protective scope of the present invention.

## Claims

1. A needle base retractable type safe self-locking type self-destruction syringe, comprising a syringe tube body (6) which is provided therein with a core bar (7) over which a piston (5) fits adjacent to the end of the core bar (7), a needle base provided in the tube journal at the end of the syringe tube body (6), a sealing ring (3) fitting over the outer wall of the needle base, **characterized in that** an interchangeable needle head being formed in the upper portion of the needle base and an elastic supporting clamping part (4) being provided in the lower cavity of the needle base and being provided with a locking engagement structure to cooperate with the end of the core bar, the locking engagement structure having a nature of elasticity, the elastic supporting clamping part (4) is provided on the outer portion with an elastic supporting position-limiting structure to cooperate with the inner wall of the inner cavity of the syringe tube body (6), the end of the core bar forming an engagement part to engage with the locking engagement structure.

2. A needle base retractable type safe self-locking type self-destruction syringe, comprising a syringe tube body (6) which is provided therein with a core bar (7) over which a piston (5) fits adjacent to the end of the core bar (7), a needle base provided in the tube journal at the end of the syringe tube body (6), a sealing ring (3) fitting over the outer wall of the needle base, **characterized in that** an elastic supporting clamping part (4) being provided in the lower cavity of the needle base and being provided with a locking engagement structure to cooperate with the end of the core bar, the locking engagement structure having a nature of elasticity, the elastic supporting clamping part (4) is provided on the outer portion with an elastic supporting position-limiting structure to cooperate with the inner wall of the inner cavity of the syringe tube body (6), the end of the core bar forming an engagement part to engage with the locking engagement structure.

3. A needle base retractable type safe self-locking type self-destruction syringe as claimed in claim 1, characterize in that the interchangeable needle head is a conical seat (2) with a guidance tooth hub which has a screwed opening able to cooperate with the injection needle (1).

4. A needle base retractable type safe self-locking type self-destruction syringe as claimed in claim 1, characterize in that an annular internal recess (22) is provided in the inner wall of the lower cavity of the needle base and an annular external recess (23) is provided outer wall of the needle base, the sealing ring (3) is provided in the annular external recess (23).

5. A needle base retractable type safe self-locking type self-destruction syringe as claimed in claim 3, characterize in that the guidance tooth hub conical seat (2) is provided on the outer wall with annular tooth hub (21) distributed evenly and annularly and with guidance function.

6. A needle base retractable type safe self-locking type self-destruction syringe as claimed in claim 1, characterize in that the elastic supporting clamping part (4) is in form of an approximate hollow cylinder and is provided in the upper portion with two symmetric elastic deformation wings (45) provided on the upper and outer edge with an annular protrusion lip (41) to cooperate with the annular internal recess (22) and between which is formed a cutout (44) under which the elastic supporting clamping part (4) is provided on the upper surface with two symmetric curved bosses (47) each of which is provided a ratchet claw (43), the two ratchet claws (3) are symmetric to each other and the extension lines of the upper ends of the two ratchet claws (43) forms an angle, the two sides of the ratchet claws (43) form gaps with the inner sides of the elastic deformation wings (45), the elastic supporting clamping part (4) is provided on the lower portion with a circular clamping plate (48) which being provided with a plurality of broken gaps (481) so that the circular clamping plate is divided into relevant separate clamping claws (42) by the broken gaps (481).

7. A needle base retractable type safe self-locking type self-destruction syringe as claimed in claim 1, characterize in that the syringe tube body (6) is provided in the inner wall on the tube journal at the end with amount of tooth sheathes (61) to cooperate with the corresponding amount of the tooth hubs (21), the tooth sheathes (61) is provided on the inner wall of the tube journal on the lower portion with an annular withdrawal prevention groove to cooperate with the clamping claw (42), the syringe tube body (6) is provided on the inner wall of the lower portion with protruded self-locking ring (63).

8. A needle base retractable type safe self-locking type self-destruction syringe as claimed in claim 3, characterize in that the engagement part at the end of the core bar (7) is an annular boss (701) whose outer diameter is larger than the distance between the top ends of the two ratchet plates (43), an annular clamping groove (702) is formed at the core bar on the lower portion of the annular boss (701) and a circular position-limiting plate (703) is formed at the core bar on the lower portion of the circular position-limiting plate (703), a piston (5) fits over the core bar (7)between the annular clamping groove (702) and the circular position-limiting plate (703).

9. A needle base retractable type safe self-locking type self-destruction syringe as claimed in claim 1, characterize in that the core bar (7) is provided reinforcement flanges (705) distributed in a cross form, one of the flanges (705) is provided with a cutout (706) provided with a protection plate (707) two ends of which are connected to points of the two inner walls of the cutout, the protection plate (707) is provided with a bending part (708) close to the middle of the protection plate (707), a protrusion (709) is formed at the bending part (708).
